# EUROPEAN PATENT APPLICATION

(11) **EP 4 321 153 A1**
(43) Date of publication of application: **14.02.2024**
(21) Application number: 22788449.1
(22) Date of filing: 13.04.2022
(51) Int. Cl.: A61K 9/20, A61K 31/454, A61P 37/00, A61P 25/00

(54) **PHARMACEUTICAL COMPOSITION COMPRISING SPHINGOSINE-1-PHOSPHATE RECEPTOR AGONIST WITH CONTROLLED PARTICLE SIZE**

(30) Priority: 14.04.2021 KR 20210048802
(71) Applicant: LG Chem, Ltd., Seoul 07336 (KR)
(72) Inventor: YUN, Duck Il, Seoul 07796 (KR); HAN, Hae Ju, Seoul 07796 (KR); MIN, Hyun Hong, Seoul 07796 (KR); KIM, Ji Young, Seoul 07796 (KR)
(74) Representative: Hoffmann Eitle
(86) International application number: PCT/KR2022/005371
(87) International publication number: WO 2022/220594

(57) **Abstract**

The present invention relates to a pharmaceutical composition comprising a sphingosine-1-phosphate receptor agonist with a controlled particle size and, more specifically, to a pharmaceutical composition comprising: as an active ingredient, 1-[1-chloro-6-(3-chloro-1-isopropyl-1H-indazole-5-ylmethoxy)-3,4-dihydro-naphthalene-2-ylmethyl]-piperidine-4-carboxylic acid of chemical formula 1 or a pharmaceutically acceptable salt thereof, and a pharmaceutically acceptable carrier, wherein the particle size d(0.9) of the active ingredient is 60 µm or less.

## Description

### TECHNICAL FIELD

The present invention relates to a pharmaceutical composition comprising a particle size-adjusted sphingosine-1-phosphate receptor agonist. More specifically, the present invention relates to a pharmaceutical composition comprising 1-[1-chloro-6-(3-chloro-1-isopropyl-1H-indazol-5-ylmethoxy)-3,4-dihydro-naphthalen-2-ylmethyl]-piperidine-4-carboxylic acid of the following Formula 1 or a pharmaceutically acceptable salt thereof as an active ingredient, and a pharmaceutically acceptable carrier, wherein a particle size d(0.9) of the active ingredient is 60 µm or less:

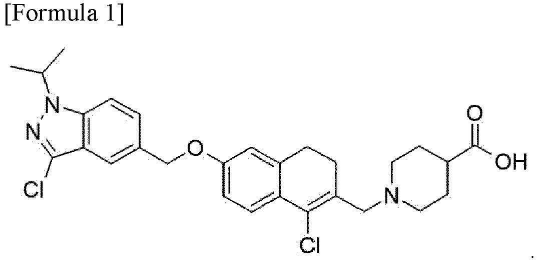

### BACKGROUND ART

Sphingosine-1-phosphate (S1P) is produced via an intracellular ceramide pathway, in which ceramide is the starting material. Ceramide is produced via two pathways, the first of which is a *de novo* biosynthetic pathway. Ceramide is also produced by the degradation of sphingomyelin, a cell membrane constituent, in a cell. The S1P level in each tissue is controlled by two biosynthetic sphingosine kinases (SphKs) and two biodegradable S1P phosphatases (S1P lyase and lysophospholipid phosphatases). S1P-which is produced via phosphorylation of sphingosine by sphingosine kinase-is known to mediate various cellular responses, such as cell proliferation, cytoskeletal organization and migration, adherence- and tight junction assembly, and morphogenesis. S1P exists as a combined form with plasma protein including albumin at high level (100 - 1,000 nM) in plasma, while it is at a low level in tissues.

S1P binds with S1P receptor, a G-protein coupled receptor, to show various biological functions. As S1P receptor sub-types, S1P1 to S1P5 are known up to now and are named endothelial differentiation gene (EDG) receptors 1, 5, 3, 6 and 8, respectively. The S1P receptors are known to be involved in various biological functions such as leukocyte recirculation, neural cell proliferation, morphological changes, migration, endothelial function, vasoregulation and cardiovascular development.

Meanwhile, in the preparation of drugs, there is a need to set the particle size specifications for the uniformity and efficacy of an active ingredient. Specifically, when the content of an active ingredient is low, it is necessary to set the particle size specifications to secure the uniformity of the active ingredient in each formulation or pharmacokinetic properties.

### DISCLOSURE OF INVENTION

### TECHNICAL PROBLEM

The present invention is intended to provide a pharmaceutical composition that can secure pharmacokinetic properties showing sufficient efficacy in which 1-[1-chloro-6-(3-chloro-1-isopropyl-1H-indazol-5-ylmethoxy)-3,4-dihydro-naphthalen-2-ylmethyl]-piperidine-4-carboxylic acid of the following Formula 1 or a pharmaceutically acceptable salt thereof is comprised with uniform content:

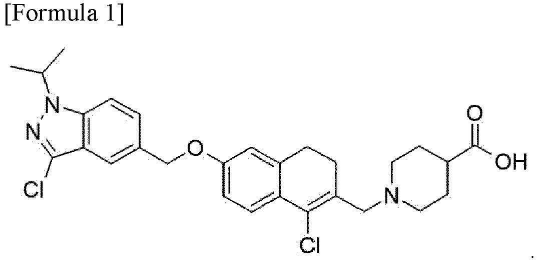

### SOLUTION TO PROBLEM

In order to solve the above technical problem, the present invention provides a pharmaceutical composition comprising 1-[1-chloro-6-(3-chloro-1-isopropyl-1H-indazol-5-ylmethoxy)-3,4-dihydro-naphthalen-2-ylmethyl]-piperidine-4-carboxylic acid or a pharmaceutically acceptable salt thereof as an active ingredient, and a pharmaceutically acceptable carrier, wherein a particle size d(0.9) of the active ingredient is 60 µm or less.

The present invention is described in detail hereinafter.

According to the present invention, there is provided a pharmaceutical composition comprising 1-[1-chloro-6-(3-chloro-1-isopropyl-1H-indazol-5-ylmethoxy)-3,4-dihydro-naphthalen-2-ylmethyl]-piperidine-4-carboxylic acid or a pharmaceutically acceptable salt thereof as an active ingredient, and a pharmaceutically acceptable carrier, wherein a particle size d(0.9) of the active ingredient is 60 µm or less.

As used herein, "particle size d(0.9)" means that 90% of the particle volume has a diameter in a specific diameter d range. Specifically, it means that the particle diameter (d(0.9)) of the point where the cumulative frequency of volume distribution reaches 90% by accumulating from the particle of the smaller particle diameter is within the range of the specific diameter d.

In one embodiment according to the present invention, to have a specific particle size, the active ingredient may be, for example micronized. In one embodiment according to the present invention, the micronization of the active ingredient may be carried out by a method known in this technical field, for example, milling.

In one embodiment according to the present invention, the lower limit of the particle size d(0.9) is not specifically limited, and may be, for example, more than 0 µm, 2 µm or more, or 5 µm or more, but is not limited thereto. In one embodiment according to the present invention, the particle size d(0.9) of the active ingredient may be 5 to 60 µm.

In one embodiment according to the present invention, the pharmaceutically acceptable salt may be selected from the group consisting of hydrochloric acid, sulfuric acid, nitric acid, phosphoric acid, hydrobromic acid, hydroiodic acid, tartaric acid, formic acid, citric acid, acetic acid, trichloroacetic acid, trifluoroacetic acid, gluconic acid, benzoic acid, lactic acid, fumaric acid, maleic acid, methanesulfonic acid, benzenesulfonic acid, p-toluenesulfonic acid and naphthalenesulfonic acid. In one embodiment according to the present invention, the pharmaceutically acceptable salt may be hydrochloric acid.

In one embodiment according to the present invention, the pharmaceutically acceptable carrier is a diluent, a binder, a lubricant and/or a disintegrant. In one embodiment according to the present invention, the diluent is lactose or a hydrate thereof, the binder is hydroxypropyl cellulose, the lubricant is sodium stearyl fumarate, and the disintegrant is croscarmellose sodium. In one embodiment according to the present invention, the hydrate of lactose is lactose monohydrate. In one embodiment according to the present invention, the pharmaceutical composition may further comprise other additives-for example, a colorant, a fragrance, a flavoring agent, a sweetener, a coating agent and the like, if necessary.

The pharmaceutical composition according to the present invention is suitable for preventing or treating diseases related to sphingosine-1-phosphate receptor. In one embodiment according to the present invention, the pharmaceutical composition may be used in the treatment of autoimmune disease including multiple sclerosis. In one embodiment according to the present invention, the pharmaceutical composition may be used in the prevention or treatment of a disease caused by undesired lymphocyte infiltration related to sphingosine-1-phosphate. In one embodiment according to the present invention, the pharmaceutical composition may be used in the prevention or treatment of immunoregulation disorder. In one embodiment according to the present invention, examples of the immunoregulation disorder may be autoimmune disease or chronic inflammatory disease selected from the group consisting of systemic lupus erythematosus, chronic rheumatoid arthritis, inflammatory bowel diseases, multiple sclerosis, amyotrophic lateral sclerosis (ALS), arteriosclerosis, atherosclerosis, scleroderma and autoimmune hepatitis, but are not limited thereto.

### EFFECTS OF THE INVENTION

The pharmaceutical composition according to the present invention can provide a medicament which contains a sphingosine-1-phosphate receptor agonist with a proper uniformity and shows sufficient efficacy.

### BRIEF DESCRIPTION OF DRAWINGS

Figure 1 is a graph showing a change in blending uniformity according to time.
Figure 2 is a graph showing comparison of *in vitro* dissolution patterns in various buffers.
Figure 3 is a graph showing comparison of pharmacokinetic property (plasma concentration) according to the particle size.

### MODE FOR THE INVENTION

Hereinafter, the present invention is explained in more detail with the following examples. However, it must be understood that the protection scope of the present invention is not limited to the examples.

### Preparation Example: Synthesis of 1-[1-chloro-6-(3-chloro-1-isopropyl-1H-indazol-5-ylmethoxy)-3,4-dihydro-naphthalen-2-ylmethyl]-piperidine-4-carboxylic acid hydrochloride

1-[1-Chloro-6-(3-chloro-1-isopropyl-1H-indazol-5-ylmethoxy)-3,4-dihydro-naphthalen-2-ylmethyl]-piperidine-4-carboxylic acid ethyl ester was synthesized according to the method described in Preparation Example 153-1 of International Publication No. WO 2014/129796 A1, the ester was hydrolyzed with NaOH, acidified with HCl, and then crystallization was carried out to obtain the hydrochloride form (hereinafter referred to as "API").

### Example 1: Preparation of tablets

After mixing the ingredients in accordance with the composition represented in Table 1 below, tablets were prepared by direct compression.

**[Table 1]**

| **Ingredients** | **Function** | **Note** | **Quantity** | | |
|---|---|---|---|---|---|
| | | | **Mg/T** | **wt%** | **g/batch** |
| API | Drug Substance | Milled | 0.5457 | 0.5457 | 1.36425 |
| Lactose Monohydrate | Diluent | SuperTab30GR | 88.4543 | 88.4543 | 221.1358 |
| Hydroxypropyl cellulose | Binder | L FP | 5.00 | 5 | 12.5 |
| Croscarmellose sodium | Disintegrant | Vivasol | 5.00 | 5 | 12.5 |
| Sodium stearyl fumarate | Lubrication | Pruv | 1.00 | 1 | 2.5 |

### Example 2: Measurement of Change in Uniformity

In order to check the change in uniformity during blending according to the particle size distribution of the active ingredient, samples having various particle distributions as represented in Table 2 below were prepared through milling of the active ingredient.

**[Table 2]**

| | Sample 1 | Sample 2 | Sample 3 | Sample 4 |
|---|---|---|---|---|
| D(0.1) [µm] | 78 | 6 | 4 | 3 |
| D(0.5) [µm] | 174 | 22 | 10 | 5 |
| D(0.9) [µm] | 309 | 60 | 19 | 9 |

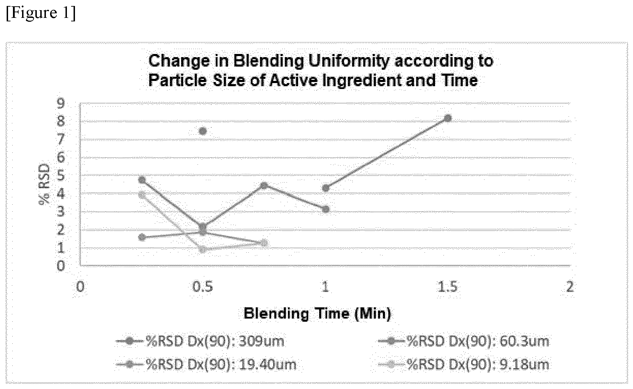

After mixing the active ingredients having different particle size distributions in Table 2 using the composition of Table 1, blending uniformity was measured, and the results are represented in Figure 1.

As can be seen from Figure 1, it could be known that the smaller the particle size, the more advantageous for uniform blending.

### Example 3: Measurement of In Vitro Dissolution Rate

Active ingredients having different particle size distributions in Table 3 below were put into gelatin capsules, and the dissolution rate according to time was measured in various pH and fasted state stimulated gastric fluid (FaSSGF) and fasted state stimulated intestinal fluid (FaSSIF, V2).

**[Table 3]**

| | Sample 5 | Sample 6 | Sample 7 |
|---|---|---|---|
| D(0.1) [µm] | 73.9 | 5.6 | 2.6 |
| D(0.5) [µm] | 176 | 22.4 | 6.4 |
| D(0.9) [µm] | 297 | 60.3 | 15.8 |

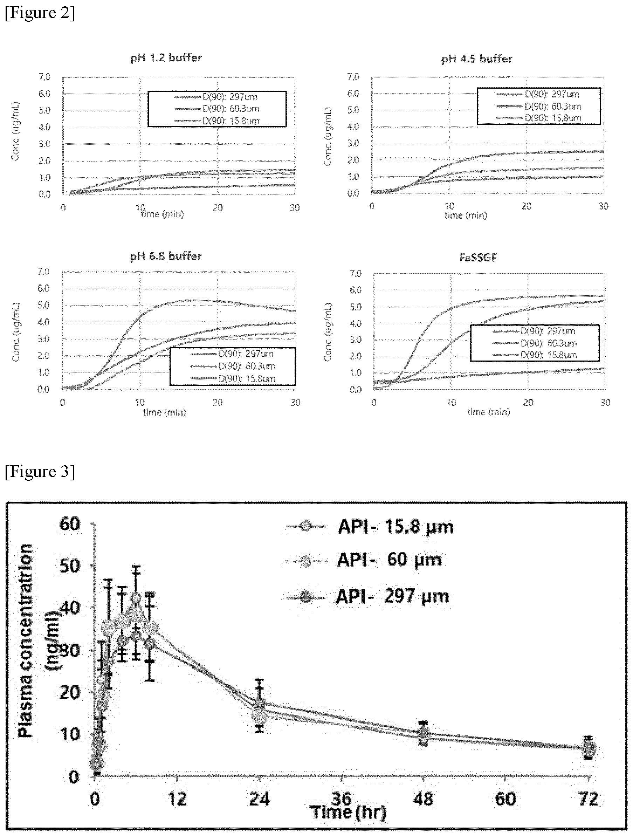

The results are represented in Figure 2. From Figure 2, although there is a difference in the degree depending on the conditions of the solution, it was confirmed that there is a difference in the dissolution rate according to the particle size under *in vitro* conditions.

### Example 4: Measurement of Pharmacokinetic Property

A crossover design of taking all three tablets (active ingredients having a particle size of Samples 5 to 7) was applied to this experimentation, and the three tablets were administered to male beagle dogs once each with a 14-day wash-out period. Each dose was taken with 50 mL of water while maintaining the fasted state for at least 14 hours. The results are represented in Figure 3.

As can be seen from Figure 3, it was confirmed that there is a difference in pharmacokinetic (PK) characteristics depending on the API particle size, and it was determined that the particle size d(0.9) based on Cmax should be 60 µm or less to show bioequivalence in terms of pharmacokinetics, and stable efficacy.

## Claims

1. A pharmaceutical composition comprising 1-[1-chloro-6-(3-chloro-1-isopropyl-1H-indazol-5-ylmethoxy)-3,4-dihydro-naphthalen-2-ylmethyl]-piperidine-4-carboxylic acid or a pharmaceutically acceptable salt thereof as an active ingredient, and a pharmaceutically acceptable carrier, wherein a particle size d(0.9) of the active ingredient is 60 µm or less.

2. The pharmaceutical composition according to Claim 1, wherein the particle size d(0.9) of the active ingredient is 5 to 60 µm.

3. The pharmaceutical composition according to Claim 1, wherein the pharmaceutically acceptable salt is selected from the group consisting of hydrochloric acid, sulfuric acid, nitric acid, phosphoric acid, hydrobromic acid, hydroiodic acid, tartaric acid, formic acid, citric acid, acetic acid, trichloroacetic acid, trifluoroacetic acid, gluconic acid, benzoic acid, lactic acid, fumaric acid, maleic acid, methanesulfonic acid, benzenesulfonic acid, p-toluenesulfonic acid and naphthalenesulfonic acid.

4. The pharmaceutical composition according to Claim 3, wherein the pharmaceutically acceptable salt is hydrochloric acid.

5. The pharmaceutical composition according to Claim 1, wherein the pharmaceutically acceptable carrier is a diluent, a binder, a lubricant and/or a disintegrant.

6. The pharmaceutical composition according to Claim 5, wherein the diluent is lactose or a hydrate thereof, the binder is hydroxypropyl cellulose, the lubricant is sodium stearyl fumarate, and the disintegrant is croscarmellose sodium.

7. The pharmaceutical composition according to Claim 6, wherein the hydrate of lactose is lactose monohydrate.

8. The pharmaceutical composition according to Claim 1, which is for use in the treatment of autoimmune disease including multiple sclerosis.

9. The pharmaceutical composition according to Claim 1, which is for use in the prevention or treatment of a disease caused by undesired lymphocyte infiltration related to sphingosine-1-phosphate.

10. The pharmaceutical composition according to Claim 1, which is for use in the prevention or treatment of immunoregulation disorder.

11. The pharmaceutical composition according to Claim 10, wherein the immunoregulation disorder is autoimmune disease or chronic inflammatory disease selected from the group consisting of systemic lupus erythematosus, chronic rheumatoid arthritis, inflammatory bowel diseases, multiple sclerosis, amyotrophic lateral sclerosis (ALS), arteriosclerosis, atherosclerosis, scleroderma and autoimmune hepatitis.
